# EUROPEAN PATENT APPLICATION

(11) **EP 4 741 003 A2**
(43) Date of publication of application: **13.05.2026**
(21) Application number: 25212731.1
(22) Date of filing: 31.10.2025
(51) Int. Cl.: A61N 5/06

(54) **RED LIGHT THERAPY SYSTEMS FOR ABLUTIONARY ENVIRONMENTS**

(30) Priority: 06.11.2024 US 202463717219 P; 20.10.2025 US 202519363251
(71) Applicant: Kohler Co., Kohler, WI 53044 (US)
(72) Inventor: BYUN, Sujung, Kohler, WI 53044 (US); FURCOIU, Aurelian, Kohler, WI 53044 (US); JAYAKUMAR, Prasanth, Kohler, WI 53044 (US); KWACZ, Jason, Kohler, WI 53044 (US); LEE, Dongha, Kohler, WI 53044 (US); ZHANG, Weimin, Kohler, WI 53044 (US)
(74) Representative: Barker Brettell LLP

(57) **Abstract**

Provided herein is a red light therapy (RLT) system including at least one of an RLT light bulb and an RLT panel. The RLT light bulb and the RLT panel may include at least one light emitting diode (LED) for providing at least one of red and infrared light. When the LEDs are activated, the LEDs may provide light therapy to a user. As one example, the RLT system may be used in a shower, bath, and/or sauna.

## Description

### Cross-Reference to Related Applications

The present application claims priority to U.S. Provisional Patent Application No. 63/717,219, filed November 6, 2024, and U.S. Patent Application No. 19/363,251, filed October 20, 2025, the entire contents of which are hereby incorporated by reference.

### Background

Red light therapy (RLT) incorporates the use of specific wavelengths of red light to provide certain health and/or therapeutic benefits. RLT may be used to improve skin condition, relieve pain, heal wounds, and/or improve blood flow.

### Brief Description of Drawings

Fig. 1 is a perspective view of example RLT panels in a shower.
Fig. 2 is a perspective view of an example RLT panel.
Fig. 3 is a perspective view of an example RLT panel.
Fig. 4 is a perspective view of an example RLT panel.
Fig. 5 is a perspective view of an example RLT panel.
Fig. 6 is a perspective view of an example RLT bench.
Fig. 7 is a cross-sectional view of an example RLT bench.
Fig. 8 is a cross-sectional view of an example RLT bench.
Fig. 9 is a perspective view of an example RLT bathtub.
Fig. 10 is a perspective view of an example floating RLT accessory.
Fig. 11 is a perspective view of an example floating RLT accessory.
Fig. 12 is a perspective view of an example floating RLT blanket.
Fig. 13 is a perspective view of an example floating RLT blanket.
Fig. 14 is a perspective view of an example RLT bath caddy.
Fig. 15 is a perspective view of an example RLT bath caddy.
Fig. 16 is a perspective view of an example RLT bath caddy.

While the disclosure is susceptible to various modifications and alternative forms, a specific embodiment thereof is shown by way of example in the drawings and will herein be described in detail. It should be understood, however, that the drawings and detailed description presented herein are not intended to limit the disclosure to the particular embodiment disclosed, but to the contrary, the intention is to cover all modifications, equivalents, and alternatives falling within the spirit and scope of the present disclosure as defined by the appended claims.

### Detailed Description

The invention will now be described with reference to the drawing figures, in which like reference numerals refer to like parts throughout. For purposes of clarity in illustrating the characteristics of the present invention, proportional relationships of the elements have not necessarily been maintained in the drawing figures.

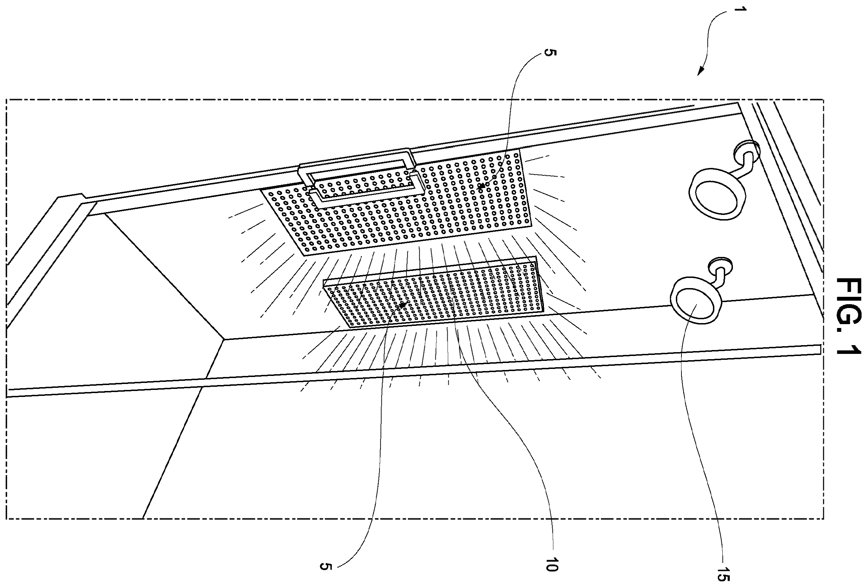

Turning first to Fig. 1, a shower 1 may include several RLT panels 5 which may be used to provide certain health and/or therapeutic benefits to a user while the user is showering. The RLT panels 5 may be any suitable shape and/or size and may include a plurality of light emitting diodes (LEDs) 10 capable of emitting red light. The LEDs 10 may be dual chip LEDs with various wavelengths (including 660 nm and 850 nm wavelengths). The LEDs 10 may be used for a specified duration and wavelength intensity. As one example, when using 660 nm and 850 nm wavelengths, the 660 nm LEDs may be used for 70-80% of the total intensity, while the 850 nm LEDs may be used for 20-30% of the total intensity. The RLT panels 5 may be positioned in the shower 1 in any suitable manner. As illustrated, the RLT panels 5 are positioned on a side of the shower 1 near showerheads 15. However, it should be understood that the RLT panels 5 may be positioned in any manner or configuration as desired by a user.

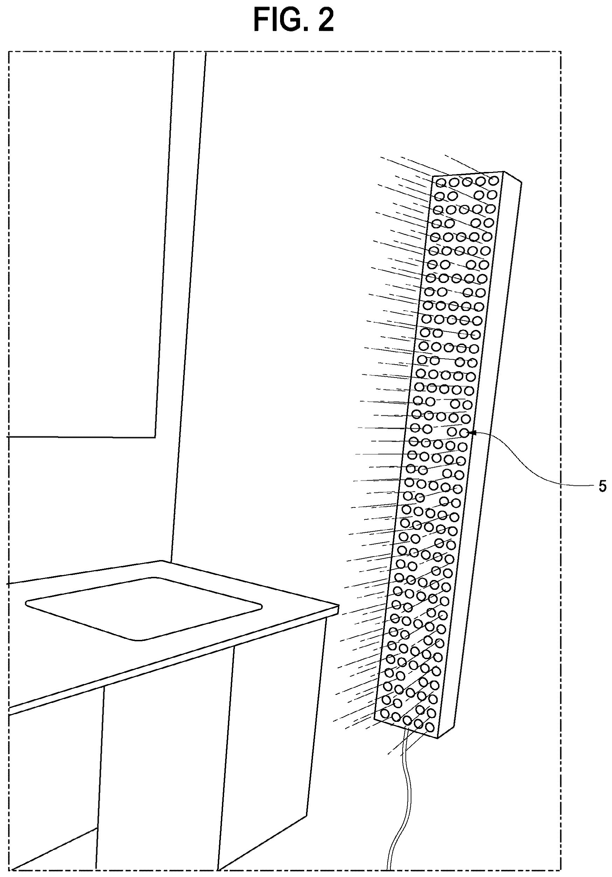

As set forth in Fig. 2, the RLT panel 5 may be placed on a bathroom wall or in any other suitable position or configuration as desired by a user. A user using a bathroom vanity or otherwise near the RLT panel 5 may use the RLT panel 5 for its health and/or therapeutic benefits.

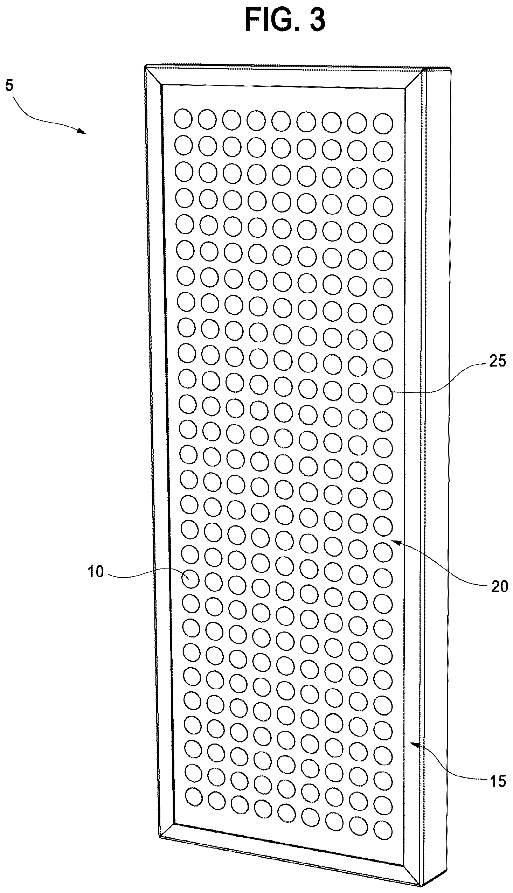

As illustrated in greater detail in Fig. 3, a front portion 15 of the RLT panel 5 may include an LED panel 20 with several LEDs 10 organized in any suitable fashion. As one example, the front portion 15 may include an outer panel 25, which may be a glass diffuser and may be made of tempered glass. As illustrated, the RLT panel 5 includes 252 LEDs 10, but other configurations may include more or fewer LEDs 10. The RLT panel 5 may be any suitable thickness, and a satisfactory thickness (e.g., 2 inches) may be selected to aid in evacuating and/or dissipating heat created by the LEDs 10. The LED panel 20 may be enclosed such that it may be substantially waterproof and may be used in wet settings (e.g., in a shower). The outer panel 25 and LED panel 20 may be encased in an enclosure, which may be made of any suitable material including aluminum.

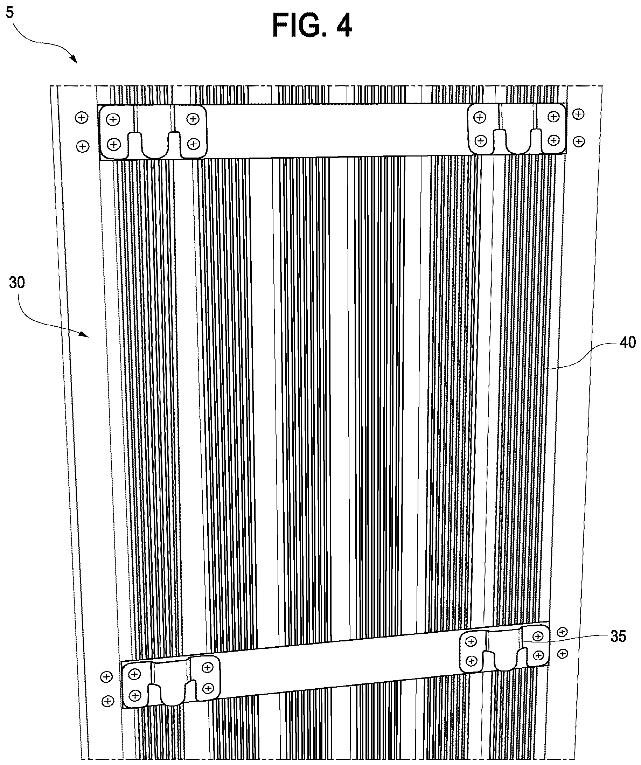

Turning to Fig. 4, a back portion 30 of the RLT panel 5 may include brackets 35 which may be used to attach the RLT panel 5 to a desired location. The brackets 35 may be any suitable bracket now known or hereafter developed. The back portion 30 of the RLT panel 5 may include several heat sinks 40, which may be used to dissipate the heat created by the LEDs 10. The heat sinks 40 may be useful to aid in ensuring that the LEDs 10 do not overheat and to increase performance of the LEDs 10. The heat sinks 40 may be any suitable heat sink now known or hereafter developed. The heat sinks 40 may be made of any suitable material, including aluminum. As illustrated, there are six heat sinks 40, but other configurations may include more or fewer heat sinks 40.

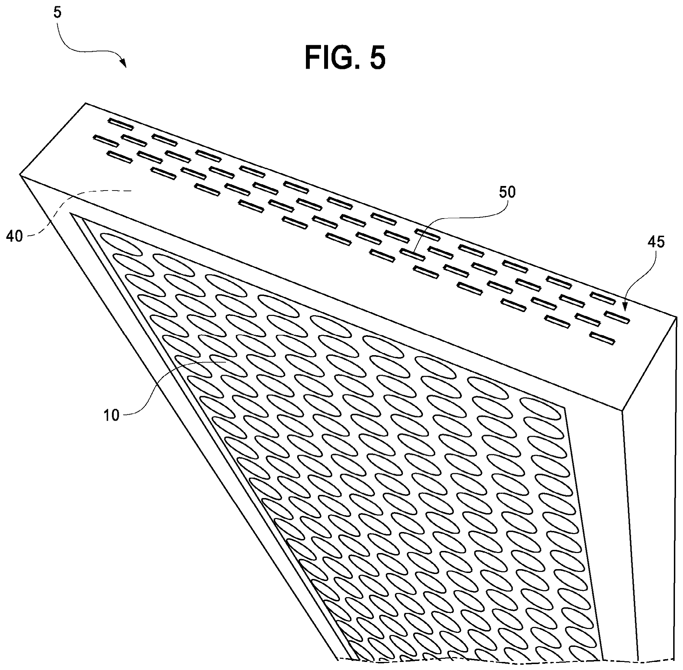

As illustrated in Fig. 5, a top portion 45 of the RLT panel 5 (which may be substantially similar to a bottom portion of the RLT panel 5 on substantially the opposite side as the top portion 45) may include several vents 50, which may allow for air or fluid (e.g., water) to enter the RLT panel 5 to aid in cooling the LEDs 10 and for heat to evacuate the RLT panel 5. The air or water that enters the RLT panel 5 via the vents 50 may enhance the capabilities of the heat sinks 40.

The RLT panel 5 may include timers such that the LEDs 10 are on for a preset period of time. The RLT panel 5 may include an "on"/"off" switch such that a user may control whether the LEDs 10 are activated. The RLT panel 5 may be powered in any suitable manner, including via a power cable and standard power source. The RLT panel 5 may be connected to other RLT panels 5 to create a larger area with the capabilities of the RLT panel 5.

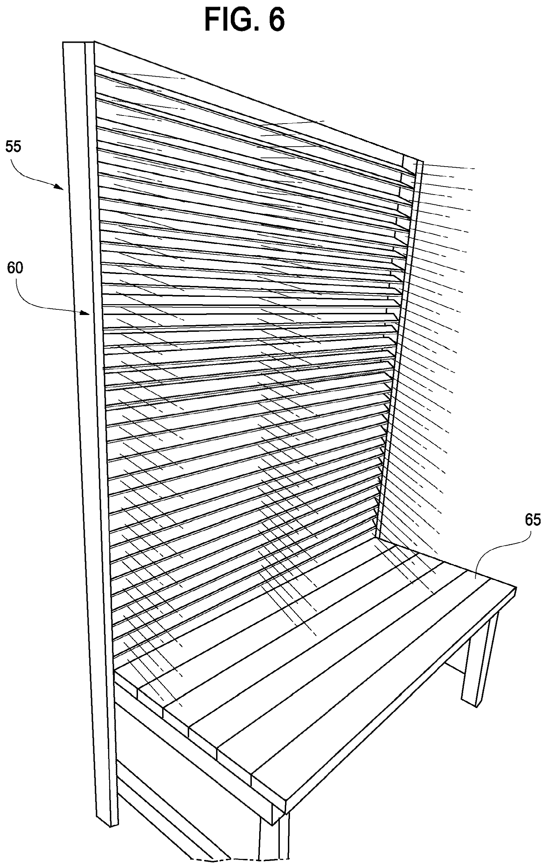

Turning now to Fig. 6, an RLT bench 55 may include an RLT wall 60 which may provide RLT to a user. As one example, a user may sit on a seat portion 65 of the RLT bench 55 to use the benefits provided by the RLT wall 60.

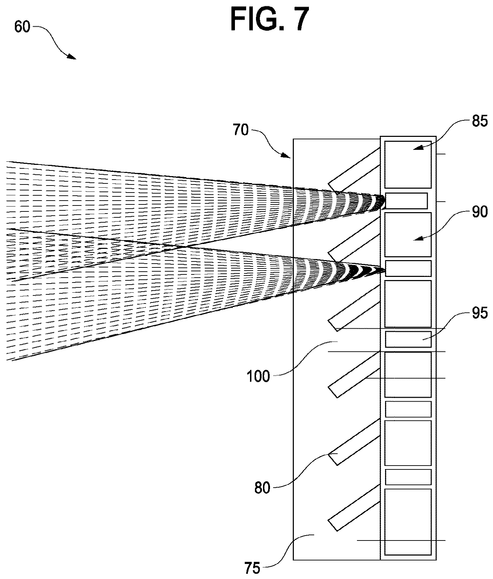

As illustrated in Fig. 7, a front portion 70 of the RLT wall 60 may include a vertical pillar 75 with slats 80 positioned thereon, and a back portion 85 of the RLT wall 60 may include an RLT panel 90. The RLT panel 90 may be similar to the RLT panel 5 as discussed with reference to Figs. 1-5. The RLT panel 90 may be any suitable shape and/or size. As one example, the RLT panel 90 may be 0.5 inches thick. The RLT panel 90 may include a plurality of LEDs 95 (which may be similar to LEDs 10 of Figs. 1-5) for providing red light to a user. The light from the LEDs 95 may extend through slots 100 formed by the space between the slats 80. The slats 80 may cause the light from the LEDs 95 to be emitted at a certain angle, including any suitable angle.

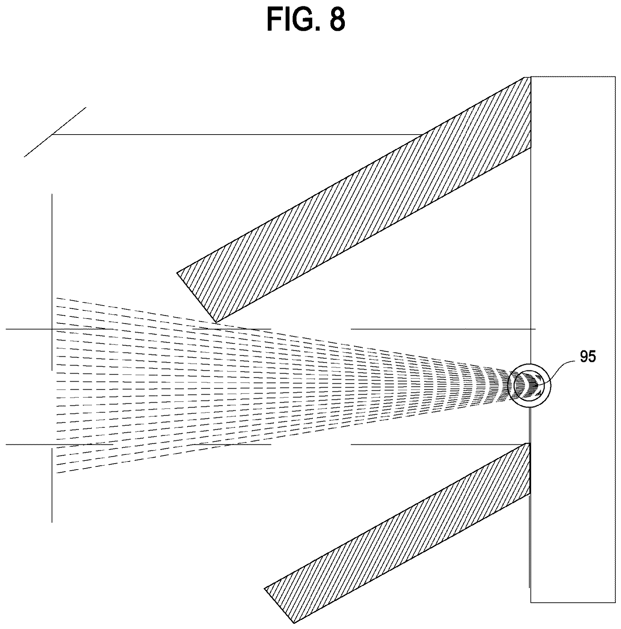

As one example, and as illustrated in Fig. 8, the angle of the light emitted from the LEDs 95 may be 22 degrees, and the light emitted from the LEDs 95 may extend 2 inches. The foregoing shall not be construed as limiting, as the angle and length of the light emitted from the LEDs 95 may be any suitable angle and/or length.

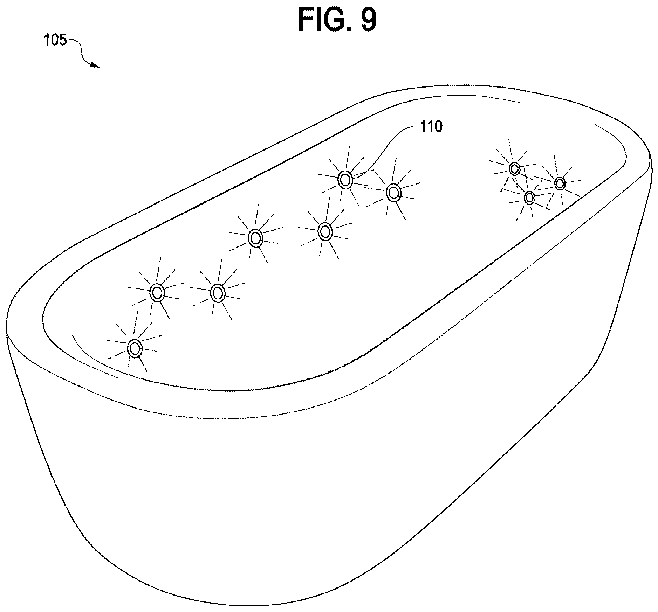

Turning to Fig. 9, a bathtub 105 may include with several RLT lights 110, which may be attached to or integrally formed with the bathtub 105. The RLT lights 110 may be spaced apart from the other RLT lights 110 in any configuration. The placement and/or pattern of the RLT lights 110 may be designed in any suitable manner to enhance a user's experience. The RLT lights 110 may be made of several LED cluster bulbs (e.g., 660 nm wavelength lights) and may include heat sinks to cool the LEDs 95. The RLT lights 110 may be effective at a suitable distance (e.g., distance of 5-30 cm). The RLT lights 110 may or may not be used and provide benefits even when not in direct contact with a user. The bathtub 105 may include timers such that the RLT lights 110 are on for a preset period of time. The bathtub 105 may include an "on"/"off" switch such that a user may control whether the RLT lights 110 are on or off.

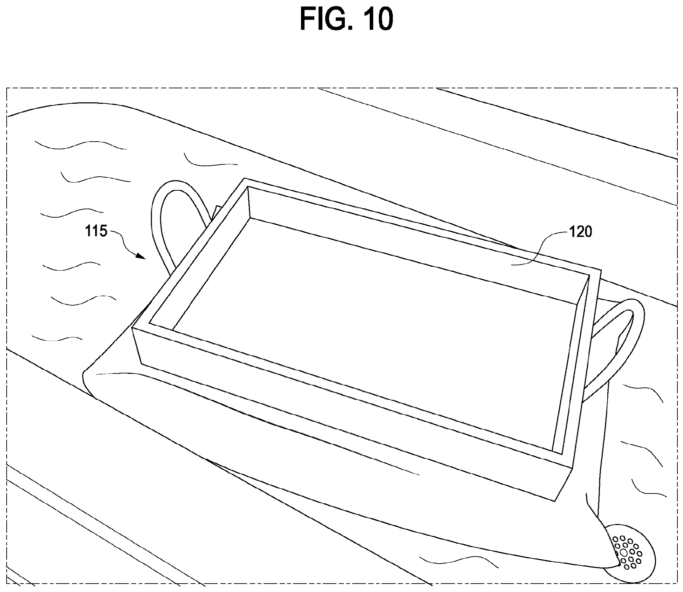

Turning now to Fig. 10, a floating accessory 115 may float such that a user may use it in a bathtub, for example. The floating accessory 115 may include a top portion 120 which may be similar to a caddy such that a user may place items in and/or on the top portion 120. The top portion 120 may be used to charge a user's phone, connect the user's phone to play music, store items, etc. The top portion 120 may include an RLT panel similar to those described herein.

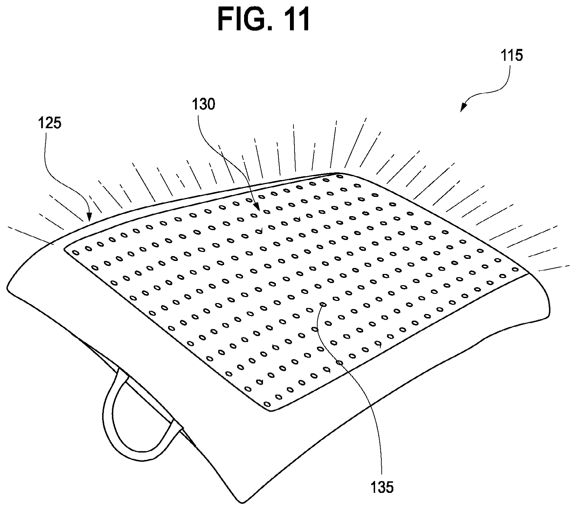

As illustrated in Fig. 11, an under portion 125 of the floating accessory 115 may cause the floating accessory 115 to float using means now known or hereafter developed. The under portion 125 of the floating accessory 115 may include an RLT panel 130 with several LEDs 135 positioned therein. The RLT panel 130 and LEDs 135 may be similar to the other panels and LEDs as discussed herein. The LEDs 135 may emit red light, and when the LEDs 135 are turned on, the user may receive RLT from the RLT panel 130. The under portion 125 may be waterproof, such that the LEDs 135 may operate even when submerged or on top of water. The floating accessory 115 may include an "on"/"off" switch which may be used to activate or deactivate the LEDs 135 in the RLT panel 130.

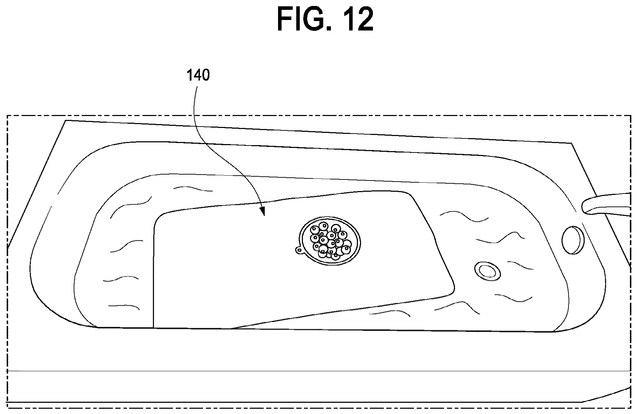

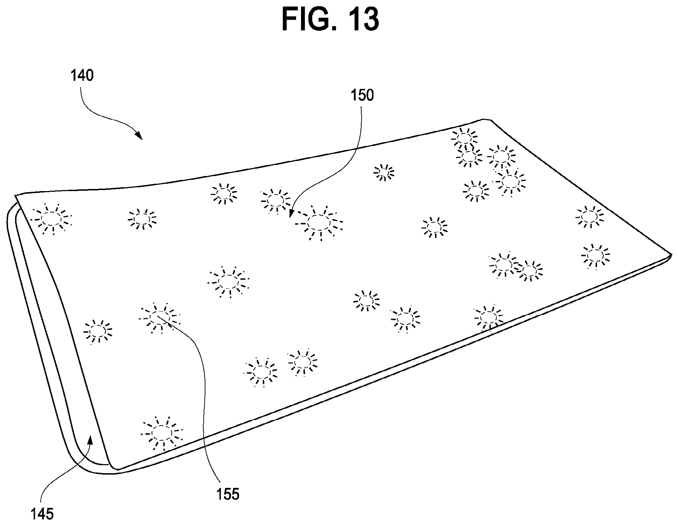

Turning to Fig. 12, a floating blanket 140 may float such that a user may use it in or outside of a bathtub, for example. As illustrated in Fig. 13, an under portion 145 of the floating blanket 140 may cause the floating blanket 140 to float using means now known or hereafter developed. The under portion 145 of the floating blanket 140 may include an RLT panel 150 with several LEDs 155 positioned therein. The RLT panel 150 and LEDs 155 may be similar to the other panels and LEDs as discussed herein. The LEDs 155 may emit red light, and when the LEDs 155 are turned on, the user may receive RLT from the RLT panel 150. The under portion 145 may be waterproof, such that the LEDs 155 may operate even when submerged or on top of water. The floating blanket 140 may be used outside of the bath, and a user may enjoy the benefits of the RLT panel 150 when laying down on the couch or in the sauna, for example. The floating blanket 140 may include an "on"/"off" switch which may be used to activate or deactivate the LEDs 155 in the RLT panel 150.

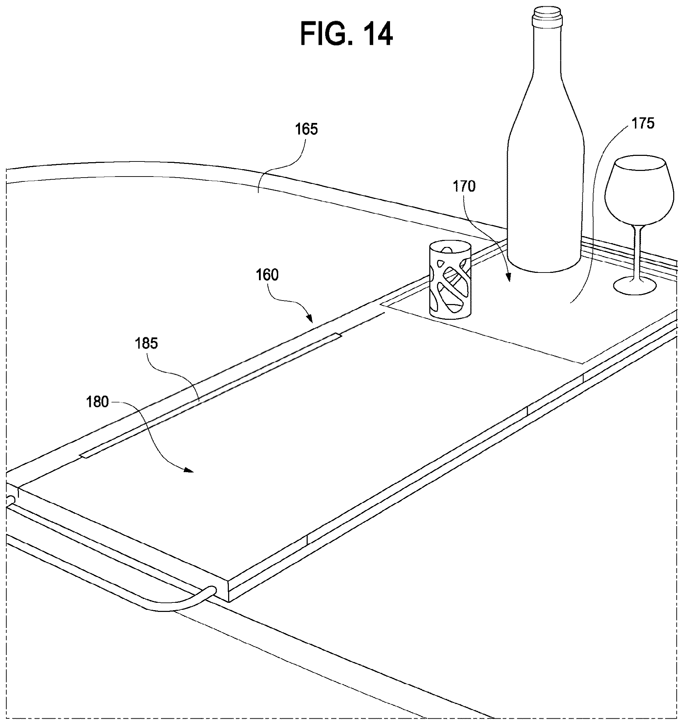

Turning now to Fig. 14, an RLT bath caddy 160 may be designed such that it may be placed on a bathtub 165, for example. The RLT bath caddy 160 may be made of any suitable material, including plastic, glass, etc. An upper portion 170 of the RLT bath caddy 160 may include a shelf portion 175, which a user may store items on. The shelf portion 175 may be used to charge a user's phone, connect the user's phone to play music, store items, etc. The upper portion 170 may include a moveable panel 180 which may be attached to the upper portion 170 via a hinge 185 or other suitable mechanism.

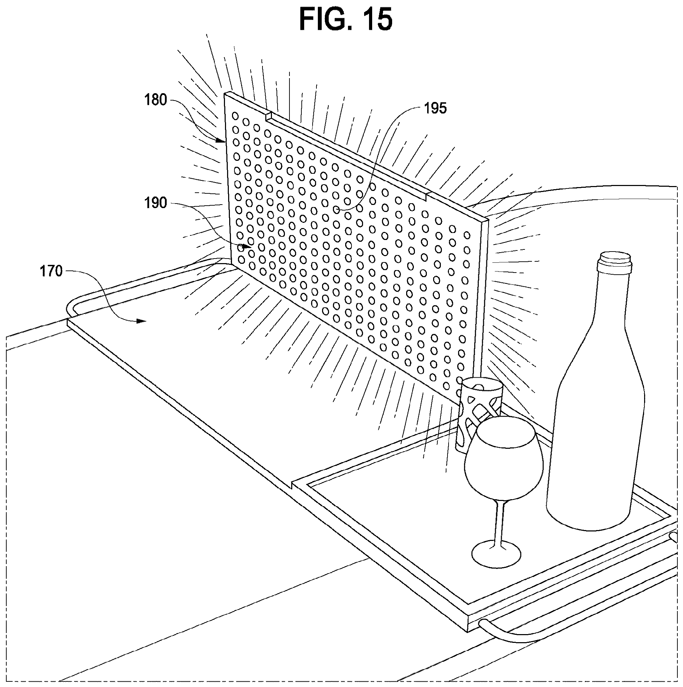

As illustrated in Fig. 15, the moveable panel 180 may hinge about the upper portion 170 to expose an underside 190 of the moveable panel 180. The underside 190 may include several LEDs 195 which may provide red light or other light therapy. The underside 190 may be designed similarly to the RLT panels as described herein. The moveable panel 180 may be adjusted in any suitable manner to cause the LEDs 195 to face in a desirable direction. As illustrated, the moveable panel 180 may hinge approximately 90 degrees, and when adjusted, the moveable panel 180 may provide RLT to a user. The RLT may be useful for a user's skin on his face or neck to reduce fine lines and wrinkles, increase blood circulation, and/or promote wound recovery. The moveable panel 180 may rotate such that it may face the opposite direction as the direction illustrated by Fig. 15. Even when the moveable panel 180 is lowered (as illustrated in Fig. 14), the LEDs 195 may be turned on and, as one example, when the upper portion 170 is made of a see-through material, the LEDs 195 may shine downward into the bathtub 165.

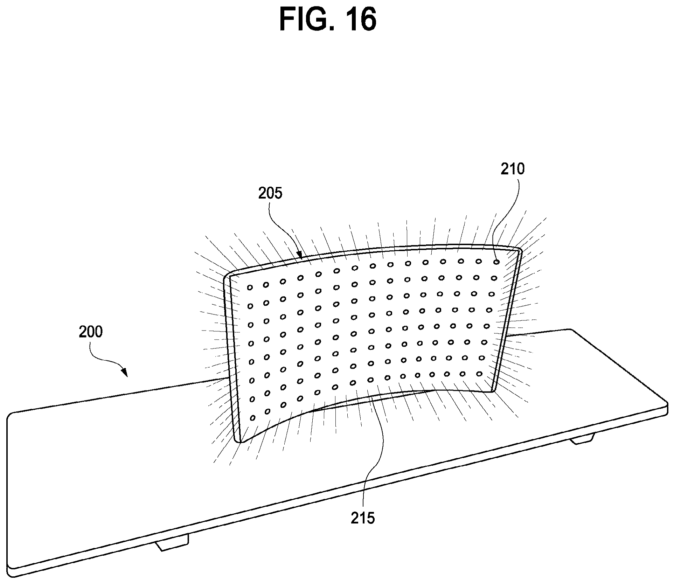

Turning to Fig. 16, a bath caddy 200 (or other movable table) may include a removable RLT panel 205. As one example, the bath caddy 200 may be positioned on top of a bathtub such that a user may use the bath caddy 200 while in the bathtub. As another example, the bath caddy 200 may be placed in any other suitable region (e.g., on a table near the bathtub). The removable RLT panel 205 may be similar to the RLT panels as described herein and may include several LEDs 210 which may be similar to the LEDs as described herein. The removable RLT panel 205 may be received via a panel holder 215 on the bath caddy 200, such that it may be rotated and adjusted in any suitable manner as desired by a user.

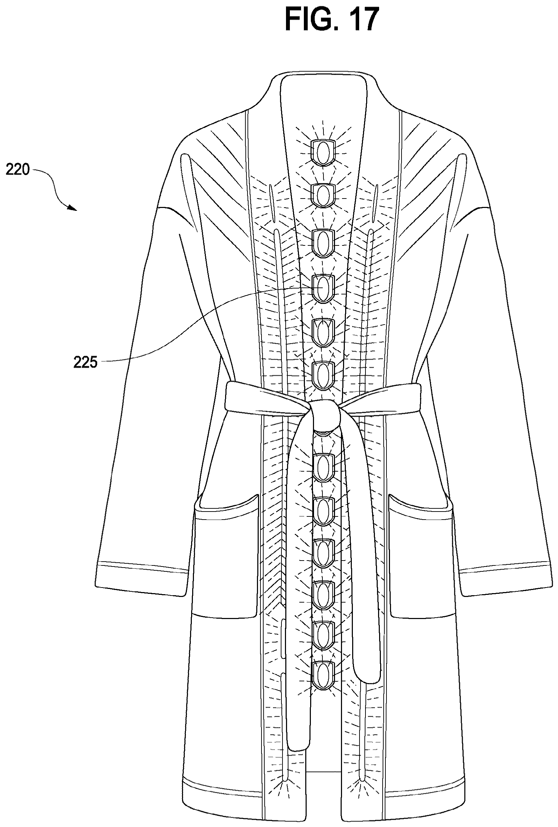

The RLT technology as described herein may be used in various capacities. As illustrated in Fig. 17, a robe 220 may include several LEDs 225 (which may be similar to the LEDs as described herein). The LEDs 225 may provide a user with RLT when a user is wearing the robe 220. As another example, several LEDs may be incorporated into a towel, clothing item, and/or apron to provide a user with RLT.

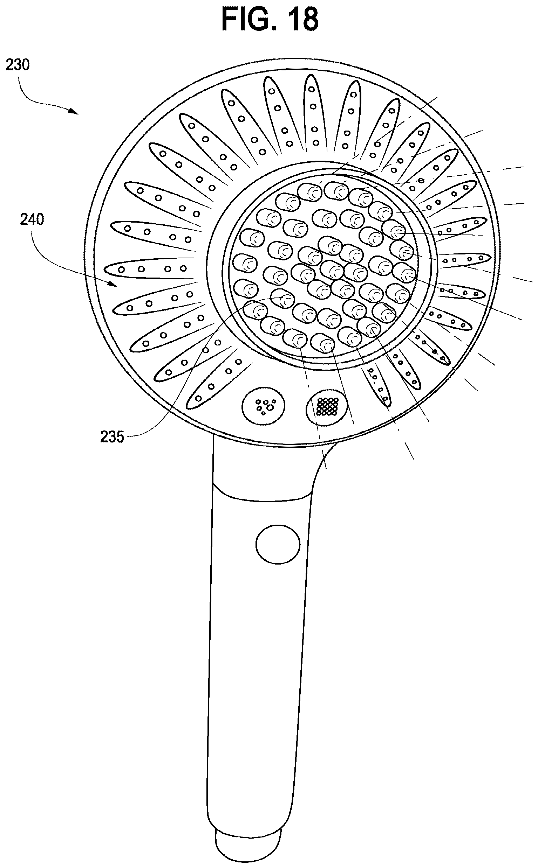

Turning to Fig. 18, a showerhead 230 may include several LEDs 235 (which may be similar to the LEDs as described herein) on a sprayplate 240. The LEDs 235 may be organized in any suitable manner on the sprayplate 240, including in the manner as illustrated in Fig. 18. The LEDs 235 may be directed toward a user when the showerhead 230 is in use and may provide RLT to the user. As further examples, LEDs for providing RLT may be incorporated into a mirror and/or mirror cabinet, a lamp used in a sauna, a folding panel, a head rest (which may be used in various places, including in a bathtub), and/or a toilet lid.

The present disclosure is disclosed in the context of shower systems but not limited to shower systems. Aspects of the present disclosure that are depicted in the illustrated embodiments or otherwise described herein may be used in conjunction with other ablutionary fittings or water distribution systems. Water distribution systems encompassed by the present disclosure include, without limitation, water distribution systems that dispense water for consumption and/or washing and water distribution systems used for private, public, domestic, residential, commercial, and/or industrial use. Water distribution systems such as, for example and without limitation, showers, baths, washtubs, hot tubs, sinks, fountains, water dispensers, and the like may incorporate aspects of the present disclosure and are encompassed herein. Example water distribution systems may include an outlet dispensing water or other fluid. The outlet may include any suitable device that is configured to dispense liquid or water. The outlet may include an ablutionary fitting, such as, for example and without limitation, a showerhead, shower spray, wand hand shower, faucet, wand, spigot, tap, spout, or the like. The outlet can include a single outlet or more than one outlet. Where the outlet includes multiple, e.g., two or more outlets, the outlets can be similar types of outlets or dissimilar types of outlets. Elements and features described with reference to one illustrated embodiment are not limited to that embodiment only; the features and elements of any one or more of the illustrated embodiments can be utilized in any other embodiment in any combination.

As is evident from the foregoing description, certain aspects of the present invention are not limited by the particular details of the examples illustrated herein, and it is therefore contemplated that other modifications, applications, variations, or equivalents thereof, will occur to those skilled in the art. Many such changes, modifications, variations, and other uses and applications of the present constructions will, however, become apparent to those skilled in the art after considering the specification and the accompanying drawings. In addition, unless mention was made above to the contrary, it should be noted that all of the accompanying drawings are not to scale. All such changes, modifications, variations, and other uses and applications which do not depart from the spirit and scope of the present inventions are deemed to be covered by the inventions which are limited only by the claims which follow.

## Claims

1. A red light therapy (RLT) system for use in a shower system comprising at least one of:
an RLT light bulb comprising at least one light emitting diode (LED) for providing at least one of red and infrared light; and
an RLT panel comprising at least one LED for providing at least one of red and infrared light;
wherein, when the at least one LED is activated, the at least one LED provides light therapy to a user.

2. The RLT system of claim 1, further comprising one of:
a shower, wherein the RLT panel is positioned on a shower wall of the shower; or
a bench having a slatted wall, wherein the RLT panel is placed behind the slatted wall such that light from the at least one LED emits through a plurality of slats on the slatted wall.

3. The RLT system of claim 1, further comprising one of:
a bathtub, wherein at least one RLT light bulb is positioned in the bathtub;
a floating blanket, and wherein the RLT panel is positioned on an underside of the floating blanket;
a floating accessory, and wherein the RLT panel is positioned on an underside of the floating accessory; or
a bath caddy having a moveable panel, wherein the RLT panel is positioned on the moveable panel such that the at least one LED on the RLT panel is directed in a direction selected by the user.

4. The RLT system of claim 1, claim 2 or claim 3, wherein the RLT panel comprises:
a front portion comprising:
an LED panel including the at least one LED; and
an outer panel;
a back portion comprising at least one heat sink.

5. The RLT system of claim 4, wherein the LED panel and outer panel are encased in an enclosure and/or wherein the front portion is substantially waterproof.

6. The RLT system of any one of the preceding claims, wherein the RLT panel comprises a plurality of vents configured to deliver at least one of air and water to the RLT panel and/or wherein the RLT panel has a thickness of at least 2 inches.

7. A red light therapy (RLT) system for use in a shower system comprising:
an RLT panel comprising:
at least one light emitting diode (LED) for providing at least one of red and infrared light; and
at least one heat sink;
a shower, wherein the RLT panel is positioned on a shower wall of the shower;
wherein, when the at least one LED is activated, the at least one LED provides light therapy to a user.

8. The RLT system of claim 7, wherein the RLT panel comprises:
a front portion comprising:
an LED panel including the at least one LED; and
an outer panel;
a back portion comprising the at least one heat sink.

9. The RLT system of claim 8, wherein the LED panel and outer panel are encased in an enclosure and/or wherein the front portion is substantially waterproof.

10. The RLT system of claim 7, claim 8 or claim 9, wherein the RLT panel comprises a plurality of vents configured to deliver at least one of air and water to the RLT panel and/or wherein the RLT panel has a thickness of at least 2 inches.

11. A red light therapy (RLT) bench comprising:
a seat portion; and
an RLT wall comprising:
at least one light emitting diode (LED) for providing at least one of red and infrared light; and
at least one heat sink;
wherein, when the at least one LED is activated, the at least one LED provides light therapy to a user.

12. The RLT bench of claim 11, wherein the RLT wall comprises:
a front portion comprising:
a vertical pillar; and
a plurality of slats positioned on the vertical pillar;
a back portion comprising an RLT panel including the at least one LED.

13. The RLT bench of claim 12, wherein, when the at least one LED is activated, light from the at least one LED extends through slots formed by spaces between the plurality of slats.

14. The RLT bench of claim 13, wherein an angle of light extending from the at least one LED is 22 degrees and/or wherein the light from the at least one LED extends no less than 2 inches from the front portion of the RLT wall.

15. The RLT bench of claim 12, claim 13 or claim 14, wherein the RLT panel has a thickness of at least 0.5 inches.
